# EUROPÄISCHE PATENTANMELDUNG

(11) **EP 1 958 600 A1**
(43) Veröffentlichungstag der Anmeldung: **20.08.2008**
(21) Anmeldenummer: 07003066.3
(22) Anmeldetag: 13.02.2007
(51) Int. Cl.: A61F 9/013

(54) **Vorrichtung zum Schneiden einer Hornhaut eines menschlichen oder tierischen Auges**

(71) Anmelder: Schwind eye-tech-solutions GmbH & Co. KG, 63801 Kleinostheim (DE)
(72) Erfinder: Jux, Egbert, 63814 Mainaschaff (DE); Geiss, Kurt, 63762 Grossostheim (DE); Bader, Thomas, Dr., 60316 Frankfurt (DE); Stenger, Stefan, 63768 Hösbach-Feldkahl (DE); Triefenbach, Nico, 63741 Aschaffenburg (DE)
(74) Vertreter: Patentanwälte Hofstetter, Schurack & Skora

(57) **Zusammenfassung**

Die Erfindung betrifft eine Vorrichtung (10) zum Schneiden einer Hornhaut (56) eines menschlichen oder tierischen Auges (58), umfassend:
- eine Positionierungsanordnung (12), mittels welcher die Vorrichtung (10) auf dem Auge (58) zu positionieren und lösbar an diesem festzulegen ist;
- eine beweglich an der Vorrichtung (10) gehaltene Schneidkopfanordnung (18), welche ein Schneidelement (20), insbesondere eine Schneidklinge, zum Schneiden der Hornhaut (56) trägt;
- eine erste Antriebseinheit (24a) mit einer ersten Antriebswelle (26a), mittels welcher die Schneidkopfanordnung (18) relativ gegenüber der Positionierungsanordnung (12) zu bewegen ist; und
- eine zweite Antriebseinheit (24b) mit einer zweiten Antriebswelle (26b), mittels welcher das Schneidelement (20) relativ gegenüber der Schneidkopfanordnung (18) zu bewegen ist,

wobei die erste und die zweite Antriebseinheit (24a, b) zumindest beim Bewegen der Schneidkopfanordnung (18) ortsfest gegenüber der Positionierungsanordnung (12) angeordnet sind.

Die Erfindung betrifft weiterhin eine Antriebsvorrichtung (22) für eine Vorrichtung (10) zum Schneiden einer Hornhaut (56) eines Auges (58).

## Beschreibung

Die Erfindung betrifft eine Vorrichtung zum Schneiden einer Hornhaut eines menschlichen oder tierischen Auges der im Oberbegriff des Patentanspruchs 1 angegebenen Art sowie eine Antriebsvorrichtung für eine Vorrichtung zum Schneiden einer Hornhaut eines menschlichen oder tierischen Auges der im Oberbegriff des Patentanspruchs 17 angegebenen Art.

Derartige Vorrichtungen bzw. Antriebsvorrichtungen werden beispielsweise bei vorbereitenden Resektionen für das sog. LASIK-Verfahren (Laser in situ Keratomileusis) verwendet. Dazu wird die Vorrichtung auf dem Auge positioniert und an diesem festgelegt. Anschließend wird mittels eines Schneidelements ein Bereich der Hornhaut des Auges eingeschnitten. Um eine schnelle Heilung gewährleisten zu können, soll der geschnittene Hornhautbereich (Flap) dabei über eine Gewebebrücke (Hinge) am Reststroma des Auges befestigt bleiben und im Anschluss an das LASIK-Verfahren in seine ursprüngliche Position zurückgeklappt werden, wo das Epithel im äußeren Bereich des Flaps selbständig innerhalb von ein bis zwei Tagen wieder verwächst. Eine derartige Vorrichtung bzw. Antriebsvorrichtung ist dabei beispielsweise bereits aus der DE 601 15 681 T2 als bekannt zu entnehmen und umfasst eine ringförmige Positionierungsanordnung, mittels welcher sie an dem Auge zu positionieren und mittels eines Unterdrucks lösbar an diesem festzulegen ist. Zum Schneiden der Hornhaut umfasst die Vorrichtung eine schiebegeführte Schneidkopfanordnung mit einer von diesem getragenen Schneidklinge. Die Vorrichtung umfasst weiterhin eine auf einem Drehzapfen gelagerte Antriebsvorrichtung mit einer ersten Antriebseinheit, mittels welcher die Schneidkopfanordnung auf einer kreisförmigen Bahn schiebegeführt entlang der Positionierungsanordnung zu bewegen ist. Zusätzlich ist eine zweite Antriebseinheit vorgesehen, die über einen exzentrischen Drehzapfen in Eingriff mit der Schneidklinge steht und diese gemäß einer linearen Hin- und Herbewegung parallel zu ihrer Schneide bewegt. Die Antriebsvorrichtung mit den beiden Antriebseinheiten erlaubt somit ein unabhängiges Bewegen von Schneidkopf und Schneidklinge, wodurch gegenüber Vorrichtungen mit nur einer Antriebseinheit eine erhöhte Variabilität des Schneidvorgangs erzielt wird.

Als nachteilig an der bekannten Vorrichtung ist jedoch der Umstand anzusehen, dass die exzentrisch gelagerte Antriebsvorrichtung zum Bewegen der Schneidkopfanordnung mit dieser mitbewegt werden muss. Dies stellt eine potentielle Gefahrenquelle dar, da der während des Schneidvorgangs wandernde Schwerpunkt der Vorrichtung vom Operator berücksichtigt und entsprechend ausgeglichen werden muss. Zudem besteht die Gefahr, dass die Antriebsvorrichtung während des Verschwenkens an Störkonturen wie beispielsweise Wangenknochen oder dem Nasen- bzw. Stirnbein anstößt und einen Fehlschnitt der Hornhaut verursacht.

Aufgabe der vorliegenden Erfindung ist es daher, eine Vorrichtung sowie eine Antriebsvorrichtung der eingangs genannten Art zu schaffen, welche ein sichereres Schneiden einer Hornhaut eines menschlichen oder tierischen Auges ermöglichen.

Die Aufgabe wird erfindungsgemäß durch eine Vorrichtung zum Schneiden einer Hornhaut eines menschlichen oder tierischen Auges mit den Merkmalen des Patentanspruchs 1 sowie durch eine Antriebsvorrichtung für eine Vorrichtung zum Schneiden einer Hornhaut eines menschlichen oder tierischen Auges mit den Merkmalen des Patentanspruchs 17 gelöst. Vorteilhafte Ausgestaltungen mit zweckmäßigen und nicht-trivialen Weiterbildungen sind in den jeweiligen Unteransprüchen angegeben, wobei vorteilhafte Ausgestaltungen der Vorrichtung - soweit anwendbar - als vorteilhafte Ausgestaltungen der Antriebsvorrichtung und umgekehrt anzusehen sind.

Eine Vorrichtung, welche ein sichereres Schneiden der Hornhaut eines menschlichen oder tierischen Auges ermöglicht, ist erfindungsgemäß dadurch geschaffen, dass die erste und die zweite Antriebseinheit zumindest beim Bewegen der Schneidkopfanordnung ortsfest gegenüber der Positionierungsanordnung, welche üblicherweise zumindest einen Saugring umfasst, angeordnet sind. Durch die ortsfeste Anordnung wird dementsprechend beim Schneiden der Hornhaut nur noch die Schneidkopfanordnung mit dem Schneidelement relativ gegenüber der Positionierungsanordnung und den beiden Antriebseinheiten bewegt. Aufgrund der vergleichsweise geringen bewegten Masse bleibt somit der Schwerpunkt der gesamten Vorrichtung während des Schneidvorgangs zumindest im Wesentlichen ortsfest. Zudem wird der jeweilige Operator nicht mehr durch die Bewegung der beiden Antriebseinheiten abgelenkt und kann sich ausschließlich auf den Schneidvorgang konzentrieren, so dass die erfindungsgemäße Vorrichtung neben einer verbesserten Handhabung auch eine deutlich erhöhte Sicherheit und Reproduzierbarkeit bietet. Die gegenüber der Positionierungsanordnung feststehenden und ortsfesten Antriebseinheiten können beim Bewegen der Schneidkopfanordnung demnach auch nicht mehr an Störkonturen anstoßen und erfordern darüber hinaus kein Mit- oder Nachführen von Kabeln, Steuerleitungen und dergleichen.

In vorteilhaften Ausgestaltungen der Erfindung ist vorgesehen, dass die erste und die zweite Antriebswelle koaxial und/oder achsparallel und/oder senkrecht gegenüber der Positionierungsanordnung angeordnet sind. Derartige Anordnungen ermöglichen einen bauraumsparenden und konstruktiv vereinfachten Aufbau der Vorrichtung, so dass sich durch die Gewichtseinsparung und die Reduzierung der Baugröße eine erhebliche Verbesserung der Handhabung bei reduzierten Herstellungskosten ergibt. Sind die Antriebswellen senkrecht gegenüber der Positionierungsanordnung angeordnet, kann zudem einfach sichergestellt werden, dass sich der Schwerpunkt der Vorrichtung jederzeit innerhalb ihrer Aufstandsfläche befindet und keine Kippmomente auftreten.

In einer weiteren vorteilhaften Ausgestaltung der Erfindung ist vorgesehen, dass die erste Antriebseinheit relativ zur Schneidkopfanordnung oberhalb der zweiten Antriebseinheit angeordnet und die erste Antriebswelle durch die zweite Antriebseinheit geführt ist. Dies erlaubt es, die Vorrichtung besonders kompakt und mit einer geringen seitlichen Ausdehnung auszubilden. Dadurch kann die Vorrichtung in einer beliebigen Orientierung auf das Auge aufgesetzt und die Schnittrichtung dementsprechend frei gewählt werden. Alternativ kann natürlich ebenfalls vorgesehen sein, dass die zweite Antriebseinheit relativ zur Schneidkopfanordnung oberhalb der ersten Antriebseinheit angeordnet und dementsprechend die zweite Antriebswelle durch die erste Antriebseinheit geführt ist.

Dabei hat es sich weiterhin als vorteilhaft gezeigt, dass die Schneidkopfanordnung um eine gegenüber der Positionierungsanordnung horizontale Achse verschwenkbar ist. Eine derartige rotierende Schnittführung ermöglicht es im Gegensatz zu einer linearen Schnittführung, dass sich der Schwerpunkt der Vorrichtung beim Bewegen der Schneidkopfanordnung nicht oder nur unwesentlich verlagert.

Um eine optimal an die jeweiligen konstruktiven Rahmenbedingungen anpassbare Übertragung und Wandlung der Drehbewegung und Drehrichtung der ersten Antriebswelle sowie des von ihr übertragenen Drehmoments gewährleisten zu können, ist in einer weiteren vorteilhaften Ausgestaltung der Erfindung eine erste Getriebeeinheit vorgesehen, über welche die Schneidkopfanordnung mit der ersten Antriebswelle gekoppelt ist.

In einer weiteren vorteilhaften Ausgestaltung der Erfindung ist vorgesehen, dass die erste Getriebeeinheit ein erstes Winkelgetriebe umfasst. Durch die in einem definierten Winkel zueinander stehenden An- und Abtriebswellen des Winkelgetriebes wird eine hohe konstruktive Variabilität erreicht, da einerseits die erste Antriebswelle bzw. die erste Antriebseinheit und die Schneidkopfanordnung in einem bestimmbaren Winkel zueinander angeordnet und andererseits sowohl verschwenkbare als auch schiebegeführte Schneidkopfanordnung mittels der ersten Antriebswelle angetrieben werden können. Das Winkelgetriebe kann dabei sowohl als mechanisches Getriebe mit form- oder kraftschlüssiger Wirkverbindung als auch als elektrisches Getriebe ausgebildet sein.

Dabei hat es sich in weiterer Ausgestaltung als vorteilhaft gezeigt, dass das erste Winkelgetriebe ein Kronenradgetriebe und/oder ein Kegelradgetriebe umfasst. Derartige Winkelgetriebe bieten beispielsweise im Gegensatz zu aus dem Stand der Technik bekannten Schneckengetrieben aufgrund der geringeren Reibung einen höheren Wirkungsgrad bei deutlich geringerer Wärmeentwicklung. Das Kronenrad- bzw. Kegelradgetriebe kann dabei sowohl gerad- als auch schrägverzahnte Zahnräder umfassen. Denkbar sind beim Kegelradgetriebe zudem auch bogenverzahnte Kegelräder. Geradverzahnte Kronenradgetriebe ermöglichen aufgrund ihrer axialen Verschiebbarkeit einen konstruktiv einfachen Längen- oder Toleranzausgleich zwischen der ersten Antriebswelle und der Abtriebswelle des Winkelgetriebes, wodurch sich weitere Vorteile ergeben.

In einer weiteren vorteilhafte Ausgestaltung der Erfindung ist vorgesehen, dass die erste Getriebeeinheit ein Harmonic-Drive- und/oder ein Cyclo-Getriebe umfasst. Derartige Vorschubgetriebe erlauben trotz ihres geringen Gewichts hohe Übersetzungen in einer Getriebestufe und können besonders kompakt und bauraumsparend ausgebildet werden. Zudem ermöglichen sie aufgrund ihrer spielfreien Kraftübertragung eine besonders exakte und reproduzierbare Bewegung der Schneidkopfanordnung. Dies ermöglicht im Gegensatz zum Stand der Technik eine elektronische Stellungs- und Ortsbestimmung des Schneidelements.

In einer weiteren vorteilhaften Ausgestaltung der Erfindung ist eine zweite Getriebeeinheit vorgesehen, über welche das Schneidelement mit der zweiten Antriebswelle gekoppelt ist. Auf diese Weise können die bereits bei der Beschreibung der ersten Getriebeeinheit erzielbaren Vorteile hinsichtlich der Übertragung und Wandlung des Drehmoments bzw. der Drehbewegung und Drehrichtungen der zweiten Antriebswelle auch zum Bewegen des Schneidelements umgesetzt werden. Dabei ist weiterhin vorgesehen, dass die zweite Getriebeeinheit ein zweites Winkelgetriebe umfasst. Auch hier können grundsätzlich kraft- oder formschlüssige mechanische sowie elektrische Winkelgetriebe mit den bereits beschriebenen Vorteilen vorgesehen sein. In einer vorteilhaften Ausgestaltung ist auch hier vorgesehen, dass das zweite Winkelgetriebe ein Kronenradgetriebe und/oder ein Kegelradgetriebe umfasst.

In weiterer Ausgestaltung der Erfindung hat es sich als vorteilhaft gezeigt, dass die zweite Getriebeeinheit eine Abtriebseinrichtung umfasst, mittels welcher eine oszillierende Bewegung des Schneidelements ausführbar ist. Durch eine oszillierende Bewegung des Schneidelements kann eine besonders exakte und zuverlässige Schnittführung gewährleistet und beispielsweise das Ausfransen oder Einreißen der Hornhaut verhindert werden. Dabei hat es sich als vorteilhaft gezeigt, dass die Abtriebseinrichtung einen mit einer Rückstellkraft beaufschlagten Kipphebel umfasst, welcher mit dem Schneidelement gekoppelt und mittels einer über die zweite Antriebswelle antreibbaren Nockenscheibe gegen die Rückstellkraft auslenkbar ist. Durch eine derartige Kopplung zwischen Nockenscheibe und Kipphebel kann eine periodisch oszillierende Schnittbewegung mit einem variabel und kostengünstig anpassbaren Amplitudenverlauf verwirklicht werden. Zum Rückstellen des ausgelenkten Kipphebels kann beispielsweise ein kostengünstiges Federelement vorgesehen sein. Auch andere Rückstellmechanismen und -elemente, wie z.B. formschlüssige Kopplungen, sind denkbar.

Eine weitere Verbesserung der Handhabbarkeit, Zuverlässigkeit und Präzision der Vorrichtung wird dadurch erzielt, dass eine Steuereinrichtung vorgesehen ist, mittels welcher zumindest die erste Antriebseinheit zu steuern ist. Mit Hilfe der Steuereinrichtung können beispielsweise die Parameter Drehgeschwindigkeit, Drehrichtung und Drehmoment zumindest der ersten Antriebseinheit gesteuert und optimal an die jeweiligen Einsatzbedingungen angepasst werden.

In einer weiteren vorteilhaften Ausgestaltung ist vorgesehen, dass die Steuereinrichtung ausgelegt ist, zumindest die erste Antriebseinheit in Abhängigkeit einer Stellung der Schneidkopfanordnung zu steuern. Dadurch kann beispielsweise ein programmierbarer Schnittverlauf mit einer definierten Abschaltung oder Umkehrung der Vorschubbewegung der Schneidkopfanordnung verwirklicht werden. Insbesondere in Kombination mit einer spielfreien ersten Getriebeeinheit kann im Gegensatz zum Stand der Technik auf einen mechanischen Anschlag verzichtet und eine signifikante Verbesserung der Schnittsicherheit und -reproduzierbarkeit gewährleistet werden. Dabei kann natürlich ebenfalls vorgesehen sein, dass auch die zweite Antriebseinrichtung mittels der Steuereinrichtung zu steuern ist.

Um eine besonders einfache und variable Einstellung der Flap-Dicke ermöglichen zu können, hat es sich weiterhin als vorteilhaft gezeigt, dass eine Einstelleinrichtung vorgesehen ist, mittels welcher die Schneidkopfanordnung in einem bestimmbaren Abstand gegenüber der Positionierungsanordnung anzuordnen ist. Dabei kann ebenfalls vorgesehen sein, dass der Einstelleinrichtung ein steuerbarer Antrieb zugeordnet ist, wodurch der Abstand zwischen Schneidkopf- und Positionierungsanordnung beispielsweise in Abhängigkeit der Stellung der Schneidkopfanordnung verändert werden kann. Dies ermöglicht komplexe Schnittformen wie beispielsweise den Schnitt einer Hornhauttasche, ovale Flaps oder Flaps mit variablen Querschnittsbereichen.

Ein weiterer Aspekt der Erfindung betrifft eine Antriebsvorrichtung für eine Vorrichtung zum Schneiden einer Hornhaut eines menschlichen oder tierischen Auges, insbesondere für ein Mikrokeratom, wobei ein sichereres Schneiden der Hornhaut erfindungsgemäß dadurch geschaffen ist, dass die erste Getriebeeinheit ein erstes Winkelgetriebe, insbesondere ein Kronenradgetriebe und/oder ein Kegelradgetriebe, umfasst. Mit Hilfe einer derartigen Getriebeeinheit kann die zugeordnete erste Antriebseinheit in einem frei bestimmbaren Winkel gegenüber der anzutreibenden Schneidkopfanordnung der Vorrichtung angeordnet werden, wodurch die Gefahr einer Kollision zwischen der Antriebsvorrichtung und besagten Störkonturen beim Bewegen der Schneidkopfanordnung ausgeschlossen werden kann. Dabei ist zu betonen, dass mittels der erfindungsgemäßen Antriebsvorrichtung sämtliche bekannten Bauformen von Schneidkopfanordnungen bewegt werden können. Weiterhin sind durch das Winkelgetriebe natürlich auch die bereits bei der im Vorhergehenden dargestellten erfindungsgemäßen Vorrichtung beschriebenen Vorteile verwirklichbar.

Dabei hat es sich in weiterer Ausgestaltung als vorteilhaft gezeigt, dass die erste Getriebeeinheit ein mit dem Winkelgetriebe gekoppeltes Harmonic-Drive- und/oder ein Cyclo-Getriebe umfasst. Eine derartige Getriebeeinheit ermöglicht bei Wirkungsgraden bis zu 90% und mehr unterschiedlichste Unter- und Übersetzungsverhältnisse, kann dabei aber wesentlich kompakter und leichter ausgebildet werden als übliche Getriebevorrichtungen. Dazu ermöglicht eine derartige Getriebeeinheit einen spiel- und wartungsfreien Antrieb und besitzt üblicherweise eine Lebensdauer von über 15000 Betriebsstunden. Aufgrund des spielfreien Antriebs können auch problemlos häufige Starts, Stops und Bewegungsumkehrungen vorgenommen werden, ohne die Zuverlässigkeit der Getriebeeinheit nachteilig zu beeinflussen.

Besonders flexible und bauraumsparend kann die Antriebsvorrichtung dadurch ausgebildet werden, dass die zweite Getriebeeinheit ein zweites Winkelgetriebe, insbesondere ein Kronenradgetriebe und/oder ein Kegelradgetriebe, umfasst. Auch hier wurden die dadurch erzielbaren Vorteile bereits zuvor beschrieben.

Um eine oszillierende Bewegung eines über die zweite Antriebseinheit bzw. Antriebswelle bewegbaren Schneidelements zu ermöglichen, ist in weiterer Ausgestaltung der Erfindung vorgesehen, dass das zweite Winkelgetriebe mit einer Nockenscheibe gekoppelt ist, mittels welcher ein mit dem Schneidelement koppelbarer Kipphebel auszulenken ist. Die Nockenscheibe kann dabei mit unterschiedlichen Konturverläufen ausgebildet werden, so dass im Zusammenspiel mit einer Drehzahl- oder Drehrichtungssteuerung der zugeordneten Antriebswelle eine flexibel anpassbare zeitliche Auslenkung des Kipphebels bzw. des mit diesem koppelbaren Schneidelements ermöglicht ist. Auch andere Kopplungsmechanismen, z.B. formschlüssige Kopplungen zur Erzeugung von oszillierenden Bewegungen der Klinge sind denkbar.

Dabei kann die Antriebsvorrichtung besonders kompakt und handlich ausgebildet werden, indem die erste und die zweite Antriebswelle koaxial und/oder achsparallel angeordnet sind. Alternativ oder zusätzlich kann weiterhin vorgesehen sein, dass die erste und die zweite Antriebseinheit koaxial angeordnet sind, wodurch weiterer Bauraum eingespart werden kann. Insbesondere im Zusammenhang mit ebenfalls koaxial angeordneten Antriebswellen kann die Antriebsvorrichtung zudem rotationssymmetrisch ausgebildet und dadurch der Schwerpunkt in die Mitte der Antriebsvorrichtung gelegt werden.

Um die Antriebseinheit vor Verschmutzung oder mechanischer Beschädigung zu schützen, hat es sich als vorteilhaft gezeigt, dass ein Gehäuse vorgesehen ist, innerhalb welchem zumindest die erste und die zweite Antriebseinheit aufgenommen sind. Es versteht sich, dass auch die Antriebswellen bzw. die Getriebeeinheiten innerhalb des Gehäuses aufgenommen sein können. Das Gehäuse kann zudem einen Griff umfassen oder selbst als Griff ausgebildet sein, wodurch die Handhabung der Antriebsvorrichtung und gegebenenfalls der Vorrichtung weiter verbessert wird.

Um eine Reinigung der Antriebsvorrichtung zu erleichtern, ist in weiterer Ausgestaltung der Erfindung vorgesehen, dass die erste und/oder die zweite Getriebeeinheit von der Antriebsvorrichtung abnehmbar ist.

Weitere Vorteile, Merkmale und Einzelheiten der Erfindung ergeben sich aus der nachfolgenden Beschreibung eines bevorzugten Ausführungsbeispiels sowie anhand der Zeichnungen, in welchen gleiche oder funktionsgleiche Elemente mit identischen Bezugszeichen versehen sind. Dabei zeigen:
- Fig. 1: eine teilgeschnittene Ansicht eines Ausführungsbeispiels der Vorrichtung von der Seite;
- Fig. 2: eine Schnittansicht der Vorrichtung gemäß Fig. 1 von vorne;
- Fig. 3: eine Detailvergrößerung der Vorrichtung gemäß Fig. 2;
- Fig. 4: eine Schnittansicht der Detailvergrößerung gemäß Fig. 3 von oben;
- Fig. 5a-c: der Bewegungsablauf der Schneidkopfanordnung der Vorrichtung gemäß Fig. 1 bis 4 beim Schneiden einer Hornhaut eines Auges in seitlicher und teilgeschnittener Ansicht.

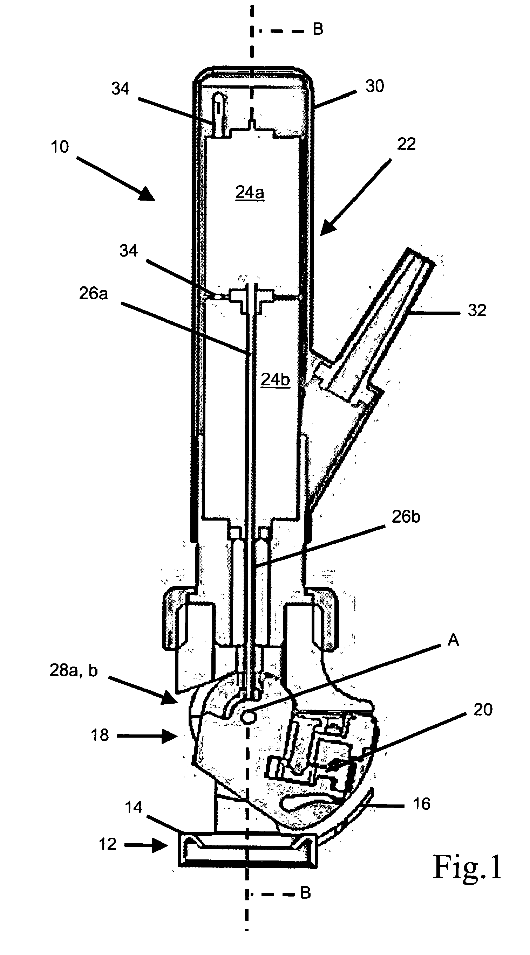

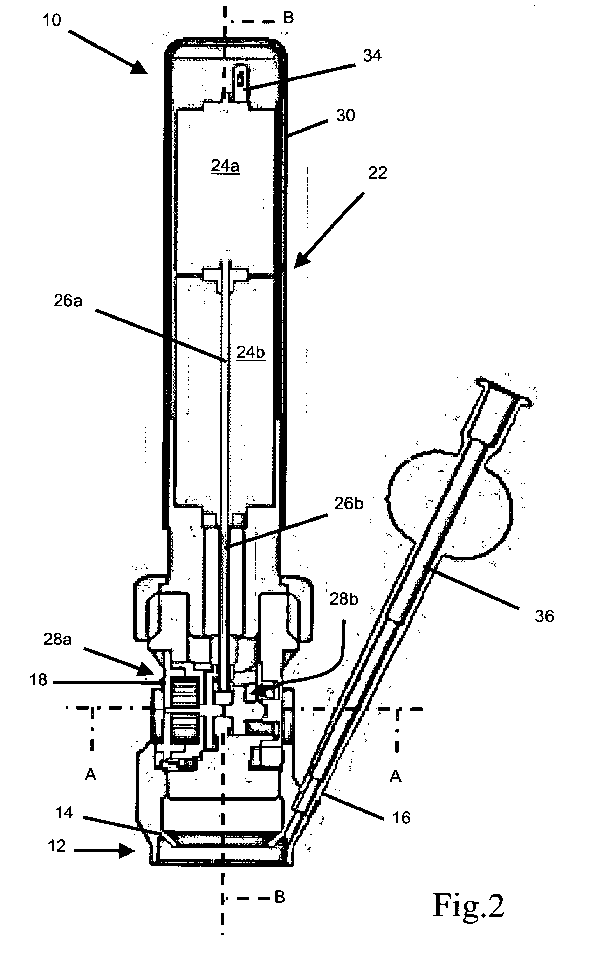

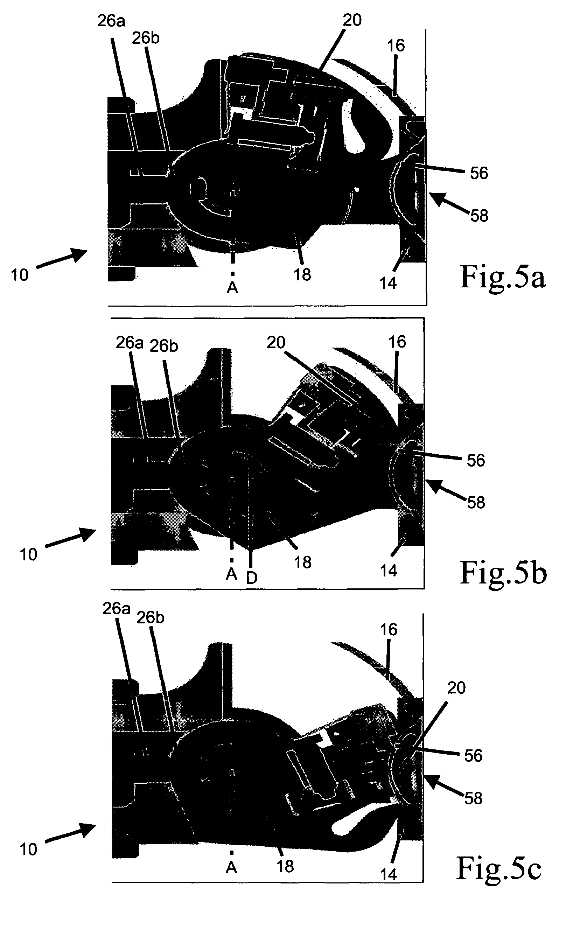

Fig. 1 zeigt eine teilgeschnittene Ansicht eines Ausführungsbeispiels der Vorrichtung 10 zum Schneiden einer Hornhaut eines menschlichen oder tierischen Auges 58 (s. Fig. 5a-c) von der Seite. Die Vorrichtung 10 umfasst eine an sich bekannte Positionierungsanordnung 12, welche einen Saugring 14 zum Positionieren der Vorrichtung 10 auf einem Auge 58 (s. Fig. 5a-c) und ein Anschlusselement 16 zum Anschließen der Positionierungsanordnung 12 an eine Pumpe (nicht abgebildet) umfasst. Auf diese Weise kann die Vorrichtung 10 durch Anlegen einer Unterdrucks lösbar am Auge 58 festgelegt werden. Weiterhin umfasst die Vorrichtung 10 eine Schneidkopfanordnung 18 mit einem als Schneidklinge ausgebildeten, bewegbaren Schneidelement 20 zum Schneiden der Hornhaut. Die gesamte Schneidkopfanordnung 18, welche im Folgenden näher erläutert werden wird, ist drehbar an der Vorrichtung 10 gehalten und um eine gegenüber der Positionierungsanordnung 12 horizontale Achse A-A verschwenkbar (s. Fig. 5a-c). Oberhalb der Schneidkopfanordnung 18 befindet sich eine gegenüber der Positionierungsanordnung 12 ortsfeste Antriebsvorrichtung 22 zum Bewegen der Schneidkopfanordnung 18 und des Schneidelements 20. Die Antriebsvorrichtung 22 umfasst ihrerseits eine erste Antriebseinheit 24a mit einer ersten Antriebswelle 26a, welche über eine erste Getriebeeinheit 28a (s. Fig. 2) mit der Schneidkopfanordnung 18 gekoppelt ist. Unterhalb der ersten Antriebseinheit 24a ist eine zweite Antriebseinheit 24b mit einer zweiten Antriebswelle 26b angeordnet, mittels welcher über eine zweite Getriebeeinheit 28b das Schneidelement 20 relativ gegenüber der Schneidkopfanordnung 18 zu bewegen ist. Die erste Antriebswelle 26a ist dabei durch die zweite Antriebseinrichtung 24b geführt und koaxial mit der zweiten Antriebswelle 26b angeordnet. Wie leicht erkennbar, sind im vorliegenden Beispiel nicht nur die Antriebswellen 26a, b sondern auch die beiden Antriebseinheiten 24a, b koaxial entlang der Achse B-B und senkrecht zur Positionierungsanordnung 12 angeordnet, wodurch sich der Schwerpunkt der gesamten Vorrichtung 10 über der Mitte der durch den Saugring 14 gebildeten Aufliegefläche befindet. Die Achse B-B stellt demzufolge gleichzeitig eine Schwerelinie der Vorrichtung 10 dar, so dass sowohl nach dem Festlegen der Vorrichtung 10 am Auge 58 als auch beim Bewegen der Schneidkopfanordnung 18 (s. Fig. 5a-c) keine Kippmomente auf die Vorrichtung 10 einwirken. Dies erlaubt ein besonders komfortables, sicheres und komplikationsfreies Schneiden einer Hornhaut 56 des Auges 58 und setzt im Gegensatz zu aus dem Stand der Technik bekannten Vorrichtungen keinen langwierigen und fehlerbehafteten Lernprozess oder außergewöhnliche chirurgische Fähigkeiten voraus. Zudem kann die Vorrichtung 10 aufgrund ihrer geringen Breite sowie ihres überwiegend rotationssymmetrischen Aufbaus frei um die Achse B-B gedreht und in der gewünschten Orientierung auf dem Auge 58 positioniert werden. Dadurch kann beispielsweise auch jede beliebige Hinge-Positionierung realisiert werden, so dass in Abhängigkeit der jeweiligen Gegebenheiten superiore, inferiore, temporale oder nasale Hinges geschnitten werden können. Aufgrund der ortsfesten Anordnung der Antriebsvorrichtung 22 besteht darüber hinaus keine Gefahr, während des Schneidens mit Störkonturen, Lidsperren, Kabeln oder dergleichen zu kollidieren.

Zur leichteren Handhabung und zum Schutz der einzelnen Bauteile vor Verschmutzung oder Beschädigung ist die gesamte Antriebsvorrichtung 22 innerhalb eines Gehäuses 30 aufgenommen, welches zusätzlich einen Griff 32 umfasst. Innerhalb des Gehäuses 30 befindet sich eine mit den Antriebseinrichtungen 24a, b gekoppelte Steuereinrichtung 34, mittels welcher die Antriebseinrichtungen 24a, b in Abhängigkeit einer Stellung der Schneidkopfanordnung 18 steuerbar ist. Die Getriebeeinheiten 28a, b sind zur Vereinfachung der Reinigung entlang der Achse A-A seitlich von der Vorrichtung 10 abnehmbar.

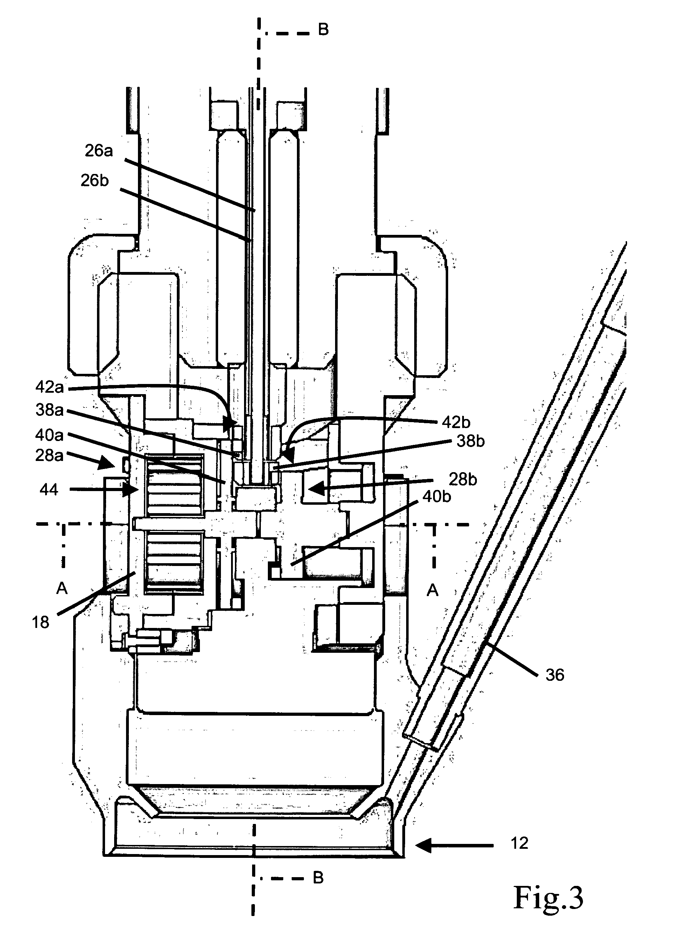

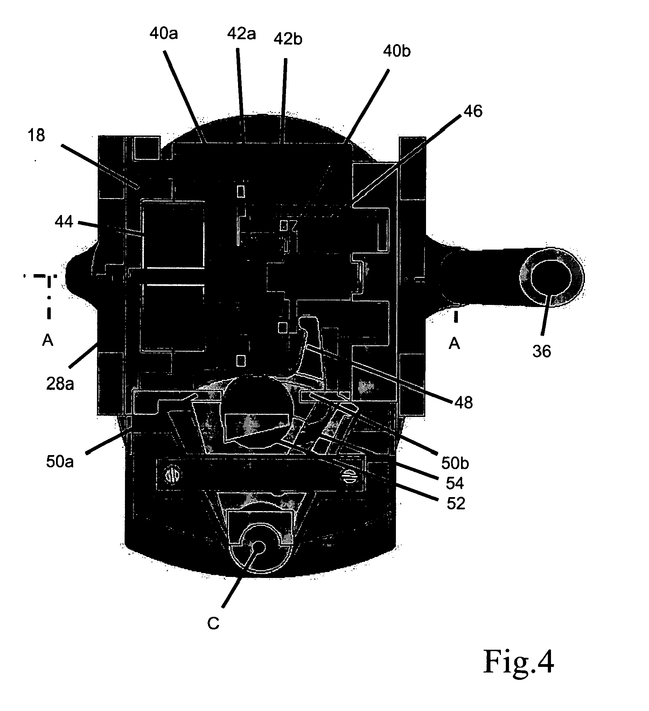

Fig. 2 zeigt eine Schnittansicht der Vorrichtung 10 gemäß Fig. 1 von vorne. Dabei ist ein mit dem Anschlusselement 16 gekoppeltes Verbindungselement 36 erkennbar, über welches die Positionierungsanordnung 12 zum Festlegen der Vorrichtung 10 am Auge 58 mit besagter Pumpe koppelbar ist. Weiterhin erkennbar sind die beiden Getriebeeinheiten 28a, b, deren Aufbau und Funktionsweise im Folgenden in Zusammenschau mit Fig. 3, welche eine Detailvergrößerung der Vorrichtung 10 gemäß Fig. 2 zeigt, und Fig. 4, welche eine Schnittansicht der Detailvergrößerung gemäß Fig. 3 von oben zeigt, näher erläutert werden soll.

Die erste Antriebswelle 26a ist über ein zylindrisches Antriebsritzel 38a formschlüssig mit einem Kronenrad 40a eines geradverzahnten Kronenradgetriebes 42a gekoppelt und treibt dieses an. Dadurch ist es möglich, die Antriebsvorrichtung 22 rechtwinklig gegenüber der Drehachse A-A der Schneidkopfanordnung 18 anzuordnen. Denkbar sind dabei jedoch auch abweichende Winkelstellungen oder die Verwendung eines Kegelradgetriebes. Das Kronenrad 40a ist seinerseits mit einem aus dem Stand der Technik bekannten Harmonic-Drive-Getriebe 44 gekoppelt, über welches der Abtrieb auf die Schneidkopfanordnung 18 erfolgt und diese um die Achse A-A verschwenkt. Durch das hohe Übersetzungsverhältnis, die Spielfreiheit und die Positionierungsgenauigkeit des Harmonic-Drive-Getriebes 44 kann die Bewegung der Schneidkopfanordnung 18 besonders exakt durchgeführt werden. Dies ermöglicht eine elektronische Stellungsbestimmung der Schneidkopfanordnung 18, so dass mittels der die erste Antriebseinrichtung 24a steuernden Steuereinrichtung 34 eine programmierbare Schnittführung ermöglicht ist. Im Gegensatz zum Stand der Technik kann daher auf eine indirekte und fehlerbehaftete Stellungsbestimmung mit Hilfe mechanischer Anschläge, Drehmomentüberwachungseinrichtungen oder dergleichen verzichtet und gleichzeitig die Genauigkeit signifikant gesteigert und der zeitliche Ablauf der Bewegung optimal an die jeweiligen Gegebenheiten angepasst werden. Dies schließt natürlich nicht aus, dass dennoch ein oder mehrere Anschläge, beispielsweise zum Kalibrieren der Schneidkopfanordnung 18, vorgesehen sein können.

Wie insbesondere in Fig. 4 erkennbar, ist die zweite Antriebswelle 26b ebenfalls über ein zylindrisches Antriebsritzel 38b formschlüssig mit einem Kronenrad 40b eines geradverzahnten Kronenradgetriebes 42b gekoppelt und treibt dieses an. Wie aus Fig. 3 und 4 deutlich wird, sind die beiden Kronenräder 40a, b koaxial entlang der Schwenkachse A-A gelagert, wobei auch alternative Anordnungen denkbar sind. Das Kronenrad 40b umfasst auf seiner dem Antriebsritzel 38b abgewandten Seite einen Nocken 46 und fungiert demnach gleichzeitig als Nockenscheibe. Alternativ kann natürlich auch vorgesehen sein, dass das Kronenrad 40b und die Nockenscheibe mehrteilig ausgebildet sind. Beim Rotieren des Kronenrads 40b läuft der Nocken 46 um die Achse A-A um und lenkt einen mit ihm gekoppelten Kipphebel 48 entsprechend dem Konturverlauf der Nockenscheibe aus. Der Kipphebel 48 ist dabei um die Achse C drehbar an der Schneidkopfanordnung 18 gelagert und trägt ein Mitnehmerelement 52, welches seinerseits mit dem Schneidelement 20 gekoppelt ist. Das Mitnehmerelement 52 überträgt demnach die Bewegung des Kipphebels 48 auf das Schneidelement 20 und bewirkt eine oszillierende Bewegung des Schneidelements 20 relativ gegenüber der Schneidkopfanordnung 18. Die Bewegung des Kipphebels 48 ist durch das Anschlagen des Mitnehmerelements 52 an den seitlichen Anschlägen 50a, b begrenzt. Der Kipphebels 48 ist weiterhin mittels eines Federelements 54 kraftbeaufschlagt und kann somit aus einer ausgelenkten Stellung in die in Fig. 4 gezeigte Stellung zurückbewegt werden. Dabei ist jedoch zu betonen, dass anstelle der beschriebenen Bewegungskopplung mittels des Kipphebels 48 auch alternative kraft- und/oder formschlüssige Kopplungstypen mit entsprechenden Bauelementen vorgesehen sein können.

Die Fig. 5a-c zeigen den Bewegungsablauf der Schneidkopfanordnung 18 der Vorrichtung 10 gemäß Fig. 1 bis 4 beim Schneiden der Hornhaut 56 des Auges 58 in seitlicher und teilgeschnittener Ansicht, wobei Fig. 5a die Startstellung der Schneidkopfanordnung 18, Fig. 5b die Mitte des Schnitts und Fig. 5c die Endstellung der Schneidkopfanordnung 18 beim Schneiden zeigt. Die Getriebeeinheiten 28a, b sind aus Gründen der Übersichtlichkeit nicht abgebildet. Das Auge 58 ist innerhalb des Saugrings 14 der Positionierungsanordnung 12 angeordnet und mittels eines über das Anschlusselement 16 aufgebrachten Unterdrucks gegenüber der Vorrichtung 10 festgelegt. Nach dem Festlegen wird zunächst das Schneidelement 20 mittels der zweiten Antriebswelle 26b in eine oszillierende Bewegung versetzt. Anschließend wird die Schneidkopfanordnung 18, angetrieben von der ersten Antriebswelle 26a, gemäß Pfeil D um die Achse A-A verschwenkt. Wie aus Fig. 5a-c erkennbar ist, bewegt sich während des Schneidens lediglich die Schneidkopfanordnung 18 mit dem Schneidelement 20, während die übrigen Bauelemente der Vorrichtung 10 und insbesondere die Antriebsvorrichtung 22 feststehen und ortsfest gegenüber der Positionierungsanordnung 12 verbleiben. Dadurch wird der Schwerpunkt der Vorrichtung 10 aufgrund der geringen bewegten Masse nicht oder nur geringfügig verändert. Dabei ist ebenfalls denkbar, dass die Schneidkopfanordnung 18 statisch bzw. dynamisch gewuchtet ist. Das in Fig. 5c gezeigte Ende des Schnitts kann - wie bereits erwähnt - aufgrund des spielfreien Antriebs über die erste Getriebeeinheit 28a mittels der als elektronischer Weggeber fungierenden Steuereinrichtung 34 ermittelt werden. Denkbar ist natürlich auch ein mechanischer Anschlag. Aufgrund der entkoppelten Bewegbarkeit von Schneidkopfanordnung 18 und Schneidelement 20 kann die Bewegung des Schneidelements 20 beim Zurückbewegen der Schneidkopfanordnung 18 entgegen Pfeil D gegebenenfalls gestoppt werden, um eine Beschädigung des geschnittenen Flaps zu verhindern. Alternativ ist es ebenfalls denkbar, dass sie Schneidkopfanordnung 18 gezielt angehalten und lediglich das Schneidelement 20 bewegt wird, so dass verschiedene Schnittformen, beispielsweise der Schnitt einer Hornhauttasche, möglich sind. Durch Einsatz einer kurvengeführten Bewegung der Schneidkopfanordnung 18 ist es ebenfalls möglich, ovale Flaps zu schneiden. Ebenso kann vorgesehen sein, dass die Vorrichtung 10 eine Höheneinstellung umfasst, mittels welcher der Abstand des Schneidelements 20 bzw. der gesamten Schneidkopfanordnung 18 gegenüber der Positionierungseinrichtung 18 veränderbar ist. Insbesondere in Kombination mit geradverzahnten Kronenradgetrieben 42 kann der dabei erforderliche Längsausgleich zwischen der Schneidkopfanordnung 18 und den Antriebswellen 26a, b problemlos bewerkstelligt werden.

## Patentansprüche

1. Vorrichtung (10) zum Schneiden einer Hornhaut (56) eines menschlichen oder tierischen Auges (58), insbesondere Mikrokeratom, umfassend:
- eine Positionierungsanordnung (12), mittels welcher die Vorrichtung (10) auf dem Auge (58) zu positionieren und lösbar an diesem festzulegen ist;
- eine beweglich an der Vorrichtung (10) gehaltene Schneidkopfanordnung (18), welche ein Schneidelement (20), insbesondere eine Schneidklinge, zum Schneiden der Hornhaut (56) trägt;
- eine erste Antriebseinheit (24a) mit einer ersten Antriebswelle (26a), mittels welcher die Schneidkopfanordnung (18) relativ gegenüber der Positionierungsanordnung (12) zu bewegen ist; und
- eine zweite Antriebseinheit (24b) mit einer zweiten Antriebswelle (26b), mittels welcher das Schneidelement (20) relativ gegenüber der Schneidkopfanordnung (18) zu bewegen ist,
**dadurch gekennzeichnet,**
**dass** die erste und die zweite Antriebseinheit (24a, b) zumindest beim Bewegen der Schneidkopfanordnung (18) ortsfest gegenüber der Positionierungsanordnung (12) angeordnet sind.

2. Vorrichtung nach Anspruch 1,
**dadurch gekennzeichnet,**
**dass** die erste und die zweite Antriebswelle (26a, b) koaxial und/oder achsparallel und/oder senkrecht gegenüber der Positionierungsanordnung (12) angeordnet sind.

3. Vorrichtung nach Anspruch 1 oder 2,
**dadurch gekennzeichnet,**
**dass** die erste Antriebseinheit (24a) relativ zur Schneidkopfanordnung (18) oberhalb der zweiten Antriebseinheit (24b) angeordnet und die erste Antriebswelle (26a) durch die zweite Antriebseinheit (24b) geführt ist.

4. Vorrichtung nach einem der Ansprüche 1 bis 3,
**dadurch gekennzeichnet,**
**dass** die Schneidkopfanordnung (18) um eine gegenüber der Positionierungsanordnung (12) horizontale Achse (A-A) verschwenkbar (Pfeil D) ist.

5. Vorrichtung nach einem der Ansprüche 1 bis 4,
**dadurch gekennzeichnet,**
**dass** eine erste Getriebeeinheit (28a) vorgesehen ist, über welche die Schneidkopfanordnung (18) mit der ersten Antriebswelle (26a) gekoppelt ist.

6. Vorrichtung nach Anspruch 5,
**dadurch gekennzeichnet,**
**dass** die erste Getriebeeinheit (28a) ein erstes Winkelgetriebe umfasst.

7. Vorrichtung nach Anspruch 6,
**dadurch gekennzeichnet,**
**dass** das erste Winkelgetriebe ein Kronenradgetriebe (42a) und/oder ein Kegelradgetriebe umfasst.

8. Vorrichtung nach einem der Ansprüche 5 bis 7,
**dadurch gekennzeichnet,**
**dass** die erste Getriebeeinheit (28a) ein Harmonic-Drive-(44) und/oder ein Cyclo-Getriebe umfasst.

9. Vorrichtung nach einem der Ansprüche 1 bis 8,
**dadurch gekennzeichnet,**
**dass** eine zweite Getriebeeinheit (28b) vorgesehen ist, über welche das Schneidelement (20) mit der zweiten Antriebswelle (26b) gekoppelt ist.

10. Vorrichtung nach Anspruch 9,
**dadurch gekennzeichnet,**
**dass** die zweite Getriebeeinheit (28b) ein zweites Winkelgetriebe umfasst.

11. Vorrichtung nach Anspruch 10,
**dadurch gekennzeichnet,**
**dass** das zweite Winkelgetriebe ein Kronenradgetriebe (42b) und/oder ein Kegelradgetriebe umfasst.

12. Vorrichtung nach einem der Ansprüche 9 bis 11,
**dadurch gekennzeichnet,**
**dass** die zweite Getriebeeinheit (28b) eine Abtriebseinrichtung umfasst, mittels welcher eine oszillierende Bewegung des Schneidelements (20) ausführbar ist.

13. Vorrichtung nach Anspruch 12,
**dadurch gekennzeichnet,**
**dass** die Abtriebseinrichtung einen mit einer Rückstellkraft beaufschlagten Kipphebel (48) umfasst, welcher mit dem Schneidelement (20) gekoppelt und mittels einer über die zweite Antriebswelle (26b) antreibbaren Nockenscheibe gegen die Rückstellkraft auslenkbar ist.

14. Vorrichtung nach einem der Ansprüche 1 bis 13,
**dadurch gekennzeichnet,**
**dass** eine Steuereinrichtung (34) vorgesehen ist, mittels welcher zumindest die erste Antriebseinheit (24a) zu steuern ist.

15. Vorrichtung nach Anspruch 14,
**dadurch gekennzeichnet,**
**dass** die Steuereinrichtung (34) ausgelegt ist, zumindest die erste Antriebseinheit (24a) in Abhängigkeit einer Stellung der Schneidkopfanordnung (18) zu steuern.

16. Vorrichtung nach einem der Ansprüche 1 bis 15,
**dadurch gekennzeichnet,**
**dass** eine Einstelleinrichtung vorgesehen ist, mittels welcher die Schneidkopfanordnung (18) in einem bestimmbaren Abstand gegenüber der Positionierungsanordnung (12) anzuordnen ist.

17. Antriebsvorrichtung (22) für eine Vorrichtung (10) zum Schneiden einer Hornhaut (56) eines menschlichen oder tierischen Auges (58), insbesondere für ein Mikrokeratom, umfassend:
- eine erste Antriebseinheit (24a) mit einer ersten Antriebswelle (26a);
- eine erste Getriebeeinheit (28a), mittels welcher die erste Antriebswelle (26a) zum Bewegen einer Schneidkopfanordnung (18) der Vorrichtung (10) mit diesem koppelbar ist;
- eine zweite Antriebseinheit (24b) mit einer zweiten Antriebswelle (26b); und
- eine zweite Getriebeeinheit (28b), mittels welcher die zweite Antriebswelle (26b) zum Bewegen eines Schneidelements (20) der Vorrichtung (10) mit diesem koppelbar ist,
**dadurch gekennzeichnet,**
**dass** die erste Getriebeeinheit (28) ein erstes Winkelgetriebe , insbesondere ein Kronenradgetriebe (42a) und/oder ein Kegelradgetriebe, umfasst.

18. Antriebsvorrichtung nach Anspruch 17,
**dadurch gekennzeichnet,**
**dass** die erste Getriebeeinheit (28a) ein mit dem ersten Winkelgetriebe gekoppeltes Harmonic-Drive- (44) und/oder ein Cyclo-Getriebe umfasst.

19. Antriebsvorrichtung nach Anspruch 17 oder 18,
**dadurch gekennzeichnet,**
**dass** die zweite Getriebeeinheit ein zweites Winkelgetriebe, insbesondere ein Kronenradgetriebe (42b) und/oder ein Kegelradgetriebe, umfasst.

20. Antriebsvorrichtung nach Anspruch 19,
**dadurch gekennzeichnet,**
**dass** das zweite Winkelgetriebe mit einer Nockenscheibe gekoppelt ist, mittels welcher ein mit dem Schneidelement (20) koppelbarer Kipphebel (48) auszulenken ist.

21. Antriebsvorrichtung nach einem der Ansprüche 17 bis 20,
**dadurch gekennzeichnet,**
**dass** die erste und die zweite Antriebswelle (26a, b) koaxial und/oder achsparallel angeordnet sind.

22. Antriebsvorrichtung nach einem der Ansprüche 17 bis 21,
**dadurch gekennzeichnet,**
**dass** die erste und die zweite Antriebseinheit (24a, b) koaxial angeordnet sind.

23. Antriebsvorrichtung nach einem der Ansprüche 17 bis 22,
**dadurch gekennzeichnet,**
**dass** ein Gehäuse (30) vorgesehen ist, innerhalb welchem zumindest die erste und die zweite Antriebseinheit (24a, b) aufgenommen sind.

24. Antriebsvorrichtung nach einem der Ansprüche 17 bis 23,
**dadurch gekennzeichnet,**
**dass** die erste und/oder die zweite Getriebeeinheit (28a, b) von der Antriebsvorrichtung (22) abnehmbar ist.
